# FASCICULE DE BREVET EUROPEEN

(11) **EP 0 714 652 B1**
(45) Date de publication et mention de la délivrance du brevet: **04.12.1996**
(21) Numéro de dépôt: 95402529.2
(22) Date de dépôt: 10.11.1995
(51) Int. Cl.: A61K 7/043

(54) **Agent de séchage rapide de film de vernis à ongles et procédé de séchage utilisant ledit agent**
Trocknungsmittel für die schnelle Trocknung von Nagellackfilmen und Verfahren zur Trocknung unter Verwendung dieses Mittels
Quick drying agent for film forming nail varnish and its process of drying by using this agent

(30) Priorité: 01.12.1994 FR 9414462
(43) Date de publication de la demande: 05.06.1996
(73) Titulaire: L'OREAL, 75008 Paris (FR)
(72) Inventeur: Ramin, Roland, F-91760 Itteville (FR)
(74) Mandataire: Dodin, Catherine

(56) Documents cités:
- WO-A-91/08731
- WO-A-92/00077
- US-A- 1 878 103
- US-A- 4 511 554
- PATENT ABSTRACTS OF JAPAN vol. 15 no. 252 (C-844) & JP-A-03 081214 (PIAS ARISE KK)

## Description

La présente invention concerne un agent de séchage rapide de film de vernis à ongles, et plus précisément un agent de séchage qui, appliqué à la surface d'une couche de vernis préalablement déposé sur l'ongle, améliore la vitesse de séchage dudit vernis à ongles.

D'une manière générale, les vernis à ongles comprennent au moins un agent filmogène, un agent plastifiant, un agent gélifiant et un solvant.
L'agent filmogène peut être de la nitrocellulose et/ou une résine telle qu'une résine alkyde, acrylique, polyuréthanne, ou résultant de la condensation de formaldéhyde avec une arylsulfonamide.
L'agent plastifiant peut être du camphre ou un phtalate.
L'agent gélifiant peut être de la bentonite, des dérivés cellulosiques et/ou des silices pyrogénées.
Le solvant peut être un alcool tel que l'éthanol, l'isopropanol ou le butanol, un acétate tel que l'acétate d'éthyle ou de butyle, une cétone et/ou un diluant tel qu'un hydrocarbure aliphatique et/ou aromatique.
Leur utilisation consiste à appliquer une ou plusieurs couches dudit vernis sur la surface de l'ongle, puis à laisser évaporer le solvant de manière à déposer un film sur l'ongle.
Les couches de vernis à ongles déposées présentent toutefois, juste après leur application, des risques de blessures par friction et/ou accrochage sur les corps étrangers. Aussi, il est souhaitable que ces vernis sèchent rapidement après avoir été appliqués.
Dans l'art antérieur, la vitesse de séchage est optimisée en sélectionnant les composants volatils, c'est à dire le solvant et éventuellement le diluant, entrant dans la composition du vernis à ongles, selon leur nature chimique et leur volatilité.
Cette optimisation a pour contrainte une limitation de la formulation du vernis à ongles, car les composants trop volatils peuvent pénaliser la qualité du maquillage obtenu. En effet, des défauts peuvent apparaître lors de l'application du vernis à ongles : formation de stries sur la couche de vernis, séchage trop rapide du pinceau lors de l'application. On obtient un film non homogène et pas suffisamment lisse; il est peu brillant et présente une tenue non satisfaisante.

Pour améliorer la qualité du maquillage des ongles, il est d'usage d'utiliser un agent de séchage rapide, ou accélérateur de séchage, qui est appliqué sur le film de vernis, en cours de séchage, pour permettre d'obtenir rapidement un film de vernis à ongles sec et dur, donc manipulable.
De tels agents de séchage sont notamment décrits dans les demandes de brevet JP-A-01016710 et JP-A-03081214 : ils comprennent au moins une huile de silicone de faible viscosité. Selon ces documents, seules les huiles de silicones de faibles viscosités, c'est à dire ayant une viscosité inférieure à 20 mPa.s, permettent d'obtenir un agent de séchage présentant de bonnes propriétés à l'application; ces huiles peuvent diffuser rapidement et uniformément à la surface du film de vernis, leur action sur le séchage du vernis est efficace et elles ne laissent pas de résidu sur le film de vernis.

Les huiles de plus hautes viscosités, c'est à dire supérieures à 20 mPa.s, ne permettent pas d'obtenir d'agent de séchage ayant de bonnes propriétés. Un étalement rapide et uniforme à la surface du film de vernis est rendu difficile par leur forte viscosité. De plus, certaines difficultés d'application peuvent se présenter lors de leur utilisation.

Pour éviter ces inconvénients, ces huiles de silicone peuvent être associées à des solvants gras ne solubilisant pas ou peu le film de vernis à ongles, tels que le myristate d'isopropyle, et/ou à des huiles minérales telles que l'huile de vaseline. Toutefois, ces solvants étant peu volatils, l'agent de séchage obtenu présente un caractère trop gras lors de son application et même après séchage.

Le but de la présente invention est de proposer un agent de séchage rapide de film de vernis à ongles pouvant comprendre des huiles de silicone de viscosité faible ou importante, permettant de déposer un film écran sur l'ongle qui limite les risques de rayures et le ternissement du vernis, sans laisser un effet gras prononcé sur l'ongle.

La présente invention a donc pour objet un agent de séchage rapide de film de vernis à ongles comprenant au moins une huile de silicone et au moins un solvant, caractérisé par le fait que ledit solvant comprend un éther de pétrole.
Un autre objet de l'invention est l'utilisation d'un éther de pétrole et d'une huile de silicone dans une composition cosmétique pouvant comprendre un solvant, notamment dans le but d'améliorer et/ou d'accélérer le séchage d'un film de vernis à ongles.

L'agent de séchage obtenu présente de bonnes propriétés cosmétiques et permet, après application sur un film de vernis, l'obtention rapide d'un film dudit vernis sec et résistant aux risques de blessures.
Un avantage de l'agent de séchage selon l'invention est qu'on peut l'appliquer sur tout type de vernis, en particulier un vernis en milieu solvant ou un vernis en milieu aqueux.

L'éther de pétrole selon la présente invention est un mélange très volatil d'hydrocarbures saturés, ramifiés ou non. Il est généralement obtenu par distillation de coupes de pétrole raffinées à des températures comprises entre 30 et 75 °C. L'éther de pétrole, connu également sous la dénomination d'essence G, est constitué essentiellement d'un mélange d'alcanes, linéaires ou ramifiés, en C5-C6, et de préférence en C5.

L'éther de pétrole peut être utilisé à une concentration comprise entre 5-99% en poids par rapport au poids total de l'agent de séchage, et de préférence à une concentration de 40-60% en poids.

L'huile de silicone selon l'invention peut être une huile de silicone ayant n'importe quelle viscosité. On peut utiliser une huile de silicone volatile ou non volatile. On peut citer, par exemple, seules ou en mélange,:
. les cyclométhicones telles que les cyclométhicones D4, D5, D6,
. les polydiméthylsiloxanes, de préférence de viscosité inférieure à 100 mPa.s,
. les polydiméthylsiloxanes-ol,
. les alkyl diméthicones répondant à la formule (I): 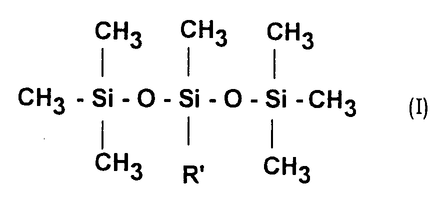
dans laquelle R' représente le radical CₙH₂ₙ₊₁, avec n = 3-8.

L'huile de silicone peut être également une huile siliconée phénylée. Cette huile peut être un polyphénylméthylsiloxane ou un phényltriméthicone, ou un mélange de différentes huiles siliconées phénylées, et en particulier peut répondre à la formule (II) suivante: 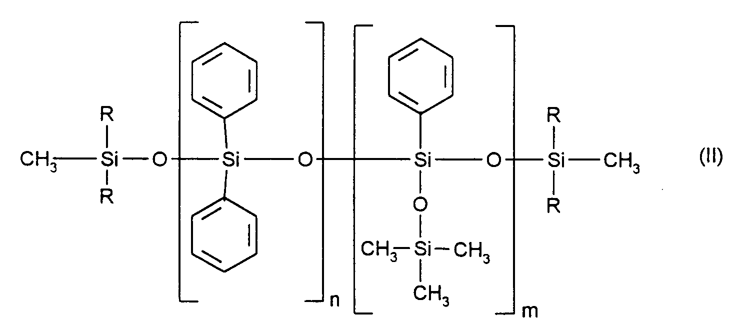 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n
est comprise entre 1 et 100.

De préférence, R est un radical méthyle, éthyle, propyle, isopropyle, décyle, dodécyle ou octadécyle, ou encore un radical phényle, tolyle, benzyle ou phénéthyle.
Parmi ces huiles phénylées, on peut citer l'huile Belsil PDM1000 de Wacker, les huiles DC556 ou SF558 de Dow Corning, l'huile Abil AV8853 de Goldschmidt, l'huile Silbione 70633V30 de Rhône Poulenc, l'huile SI 555 de la société SISS.

L'huile de silicone peut être utilisée à une teneur comprise entre 1% et 60% en poids par rapport au poids total de l'agent de séchage et de préférence entre 15-30% en poids.
On peut utiliser de préférence les huiles de silicone ayant une viscosité supérieure à 20 mPa.s, et plus préférentiellement les huiles de silicone ayant une viscosité allant de 25 mPa.s à 10 000 mPa.s.

L'agent de séchage selon l'invention peut également comprendre une gomme de silicone, éventuellement solubilisée dans une huile de silicone.
La gomme de silicone peut être, seule ou en mélange, une gomme répondant à la formule (III): 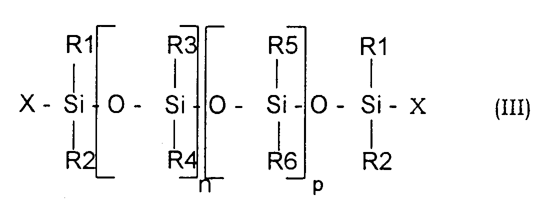 dans laquelle:
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.
Comme gomme de silicone utilisable selon l'invention, on peut citer celles pour lesquelles:
. les substituants R1 à R6 et X représentent un groupement méthyle, p = 0 et n = 2700, comme celle vendue sous la dénomination SE30 par la société General Electric,
. les substituants R1 à R6 et X représentent un groupement méthyle, p = 0 et n = 2300, comme celle vendue sous la dénomination AK 500000 par la société Waker,
. les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
. les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
. les substituants R1, R2, R5, R6 et X représentent un groupement méthyle, les substituants R3 et R4 représentent un groupement aryle tel que le poids moléculaire du composé soit de 600 000, comme celle vendue sous la dénomination 761 par la société Rhône-Poulenc.
La gomme de silicone peut être présente à une concentration de 0-10% en poids.

L'agent de séchage selon l'invention peut comprendre, en tant que solvant, d'autres composés que l'éther de pétrole. De préférence, ces composés ne doivent pas ou peu solubiliser le film de vernis déposé sur l'ongle. Ils doivent être compatibles avec l'huile de silicone, c'est à dire ne former qu'une seule phase avec l'huile de silicone utilisée.
On peut citer, par exemple, le myristate d'isopropyle, l'isobutane, l'isopropanol, l'éthanol, et leurs mélanges.

L'agent de séchage selon l'invention peut contenir, en outre, des additifs cosmétiques tels que des parfums, des colorants et/ou des pigments, ainsi que des actifs cosmétiques tels que des vitamines et/ou des filtres UV.

L'invention concerne également un procédé de séchage d'un film de vernis à ongles caractérisé par le fait que l'on applique sur le film déposé sur l'ongle un agent de séchage tel que décrit précédemment.
Ledit agent de séchage peut être appliqué de préférence à l'aide d'un pinceau ou d'un vaporisateur, ou à l'aide d'un spray aérosol.

On va maintenant donner des exemples illustrant la présente invention sans toutefois la limiter.

### Exemple 1

On prépare un agent de séchage ayant la composition suivante:

| | |
|---|---|
| . Ether de pétrole (Essence G de TOTAL) | 80 g |
| . Décaméthylcyclopentasiloxane (viscosité 4 mPa.s) | 20 g |

On a appliqué l'agent de séchage sur un film de vernis à ongles en milieu solvant. On a constaté que l'application de cet agent est facile et que le film obtenu après l'application n'est pas trop gras.

### Exemple 2

On prépare un agent de séchage ayant la composition suivante:

| | |
|---|---|
| . Ether de pétrole (Essence G de TOTAL) | 95 g |
| . Octaméthylcyclotétrasiloxane (viscosité 7 mPa.s) | 4 g |
| . Polydiméthysiloxane-ol en mélange avec des cyclotétra- et cyclopenta- diméthylsiloxanes (QC F2-1671 de DOW CORNING) | 1 g |

On a appliqué l'agent de séchage sur un film de vernis à ongles en milieu solvant. On a constaté que le film sèche rapidement et ne laisse pas d'effet gras prononcé sur l'ongle.

### Exemple 3

On prépare un agent de séchage ayant la composition suivante :

| | |
|---|---|
| . Ether de pétrole (Essence G de TOTAL) | 75 g |
| . Myristate d'isopropyle | 10 g |
| . Décaméthylcyclopentasiloxane (viscosité 4 mPa.s) | 10 g |
| . Huile de vaseline | 5 g |

On a appliqué l'agent de séchage sur un film de vernis à ongles en milieu aqueux. Le film obtenu après séchage ne présente pas de caractère gras prononcé.

### Exemple 4

On prépare un agent de séchage ayant la composition suivante :

| | |
|---|---|
| . Ether de pétrole (Essence G de TOTAL) | 67,6 g |
| . Décaméthylcyclopentasiloxane (viscosité 4 mPa.s) | 20 g |
| . Myristate d'isopropyle | 10 g |
| . Isopropanol | 2 g |
| . Filtre UV | 0,3 g |
| . Vitamines, parfum, colorant | 0,1 g |

On a appliqué l'agent de séchage sur un film de vernis à ongles en milieu aqueux. Celui-ci présente les propriétés cosmétiques attendues selon l'invention.

### Exemple 5

On prépare un agent de séchage conditionné sous forme de spray aérosol, ayant la composition suivante:

| | |
|---|---|
| . Ether de pétrole (Essence G de TOTAL) | 1,6 g |
| . Décaméthylcyclopentasiloxane (viscosité 4 mPa.s) | 1,2 g |
| . Myristate d'isopropyle | 1,2 g |
| . Isobutane | 96 g |

On a appliqué l'agent de séchage sur un film de vernis à ongles en milieu solvant. Celui-ci présente les propriétés cosmétiques attendues selon l'invention.

### Exemple 6

On prépare un agent de séchage ayant la composition suivante :

| | |
|---|---|
| . Ether de pétrole (Essence G de TOTAL) | 98 g |
| . Mélange de polyméthysiloxane-ol (14 %) et de cyclotétra-, cyclopenta diméthylsiloxane (86 %) vendu sous la dénomination QC F2-1671 par la société DOW CORNING (viscosité 7000 mPa.s) | 2 g |

On a appliqué l'agent de séchage sur un film de vernis à ongles en milieu solvant. On a constaté que le film sèche rapidement et ne laisse pas d'effet gras prononcé sur l'ongle.

## Revendications

1. Agent de séchage rapide de film de vernis à ongles comprenant au moins une huile de silicone et au moins un solvant, caractérisé par le fait que ledit solvant comprend de l'éther de pétrole.

2. Agent de séchage selon la revendication 1, dans lequel l'huile de silicone est choisie parmi les cyclométhicones, les polydiméthylsiloxanes, les polydiméthysiloxanes-ol, les silicones phénylées et les alkyl diméthicones répondant à la formule (I): 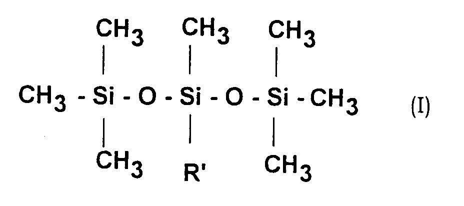 dans laquelle R' représente le radical CₙH₂ₙ₊₁, avec n = 3-8, seules ou en mélange.

3. Agent de séchage selon la revendication 2, dans lequel l'huile siliconée phénylée est choisi parmi, seuls ou en mélange, les polyphénylméthylsiloxanes, les phényltriméthicones et les huiles siliconées phénylées répondant à la formule (II) suivante : 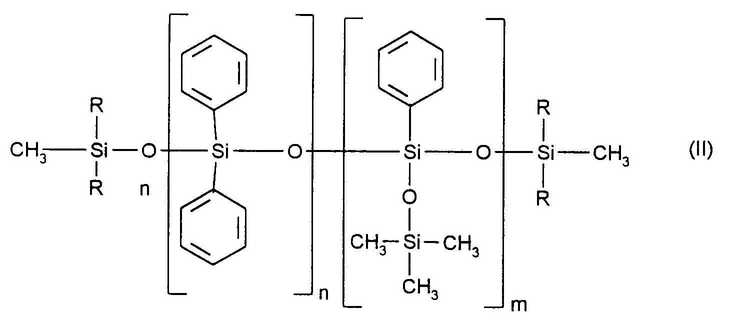 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n
est comprise entre 1 et 100.

4. Agent de séchage selon l'une quelconque des revendications précédentes, caractérisé par le fait que la teneur en huile de silicone est comprise entre 1 % et 60 % en poids par rapport au poids total de l'agent.

5. Agent de séchage selon l'une des revendications précédentes, dans lequel l'huile de silicone a une viscosité supérieure à 20 mPa.s, de préférence comprise entre 25 mPa.s et 10 000 mPa.s.

6. Agent de séchage selon l'une quelconque des revendications précédentes, caractérisé par le fait que la teneur en éther de pétrole est comprise entre 5 et 99% en poids par rapport au poids total de l'agent.

7. Agent de séchage selon l'une quelconque des revendications précédentes, caractérisé par le fait qu'il comprend en outre une gomme de silicone.

8. Agent de séchage selon la revendication 7, caractérisé par le fait que la gomme de silicone est choisie parmi, seule ou en mélange, les gommes répondant à la formule (III) : 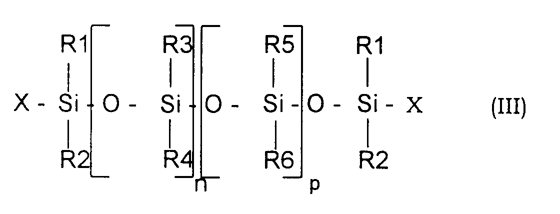 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s.

9. Agent de séchage selon l'une quelconque des revendications précédentes, caractérisé par le fait qu'il comprend au moins un autre composé que l'éther de pétrole en tant que solvant.

10. Agent de séchage selon la revendication 9, caractérisé par le fait que ledit autre composé est choisi parmi le myristate d'isopropyle, l'isobutane, l'isopropanol, l'éthanol, et leurs mélanges.

11. Agent de séchage selon l'une quelconque des revendications précédentes, caractérisé par le fait qu'il contient au moins un additif et/ou un actif cosmétique.

12. Procédé de séchage de film de vernis à ongles, caractérisé par le fait qu'on applique sur le film déposé sur l'ongle, un agent de séchage selon l'une des revendications 1 à 11.

13. Utilisation d'un éther de pétrole et d'une huile de silicone dans une composition cosmétique pouvant comprendre un solvant.

14. Utilisation selon la revendication 13, dans le but d'améliorer et/ou d'accélérer le séchage d'un film de vernis à ongles.

## Claims

1. Quick-drying agent for a film of nail varnish comprising at least one silicone oil and at least one solvent, characterized in that the said solvent comprises petroleum ether.

2. Drying agent according to Claim 1, in which the silicone oil is chosen from cyclomethicones, polydimethylsiloxanes, polydimethylsiloxanols, phenyl-containing silicones and alkyldimethicones corresponding to formula (I): 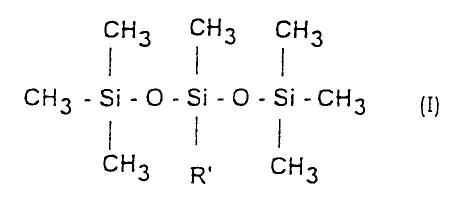 in which R' represents the radical CₙH₂ₙ₊₁, with n = 3-8, alone or as a mixture.

3. Drying agent according to Claim 2, in which the phenyl-containing silicone oil is chosen, alone or as a mixture, from polyphenylmethylsiloxanes, phenyltrimethicones and phenyl-containing silicone oils corresponding to formula (II) below 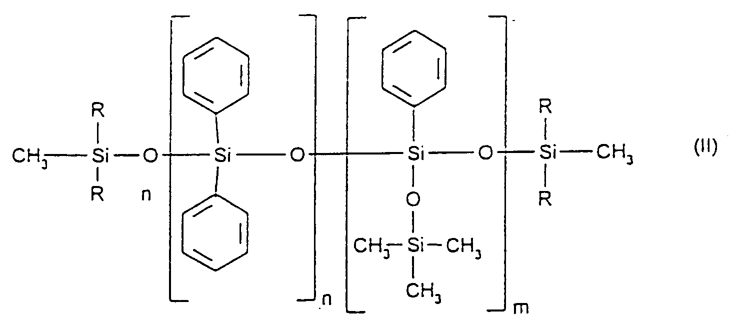 in which
• R is a C1-C30 alkyl radical, an aryl radical or an aralkyl radical,
• n is an integer between 0 and 100,
• m is an integer between 0 and 100, with the proviso that m + n is between 1 and 100.

4. Drying agent according to any one of the preceding claims, characterized in that the content of silicone oil is between 1 % and 60 % by weight relative to the total weight of the agent.

5. Drying agent according to one of the preceding claims, in which the silicone oil has a viscosity of greater than 20 mPa·s, preferably between 25 mPa·s and 10,000 mPa·s.

6. Drying agent according to any one of the preceding claims, characterized in that the content of petroleum ether is between 5 and 99 % by weight relative to the total weight of the agent.

7. Drying agent according to any one of the preceding claims, characterized in that it additionally comprises silicone gum.

8. Drying agent according to Claim 7, characterized in that the silicone gum is chosen, alone or as a mixture, from the gums corresponding to formula (III): 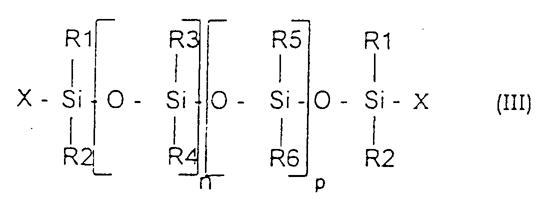 in which:
R₁, R₂, R₅ and R₆ are, together or separately, an alkyl radical having 1 to 6 carbon atoms,
R₃ and R₄ are, together or separately, an alkyl radical having from 1 to 6 carbon atoms, or an aryl radical,
X is an alkyl radical having from 1 to 6 carbon atoms, a hydroxyl radical or a vinyl radical,
n and p being chosen so as to impart a viscosity of greater than 100,000 mPa·s to the silicone gum.

9. Drying agent according to any one of the preceding claims, characterized in that it comprises at least one compound other than petroleum ether as solvent.

10. Drying agent according to Claim 9, characterized in that the said other compound is chosen from isopropyl myristate, isobutane, isopropanol, ethanol, and mixtures thereof.

11. Drying agent according to any one of the preceding claims, characterized in that it contains at least one cosmetic additive and/or one cosmetic active agent.

12. Process for drying a film of nail varnish, characterized in that a drying agent according to one of Claims 1 to 11 is applied to the film deposited on the nail.

13. Use of a petroleum ether and a silicone oil in a cosmetic composition which may comprise a solvent.

14. Use according to Claim 13, with the aim of improving and/or accelerating the drying of a film of nail varnish.

## Patentansprüche

1. Trocknungsmittel für die schnelle Trocknung von Nagellackfilmen, das mindestens ein Siliconöl und mindestens ein Lösungsmittel enthält,
**dadurch gekennzeichnet, daß**
das Lösungsmittel Petrolether enthält.

2. Trocknungsmittel nach Anspruch 1, in dem das Siliconnöl, allein oder im Gemisch, unter Cyclometiconen, Polydimethylsiloxanen, Polydimethylsiloxanolen, phenylgruppenhaltigen Siliconen und Alkyldimeticonen der Formel (I) 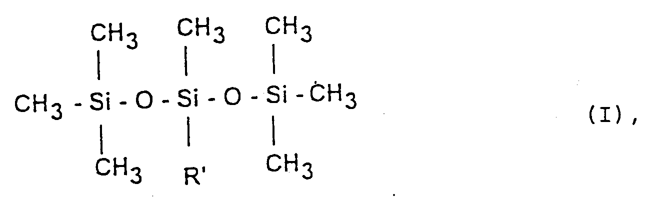 worin R' die Gruppe CₙH₂ₙ₊₁ bedeutet, in der n eine Zahl im Bereich von 3 bis 8 ist, ausgewählt ist.

3. Trocknungsmittel nach Anspruch 2, in dem das phenylgruppenhaltige Siliconöl, allein oder im Gemisch, unter Polyphenylmethylsiloxanen, Phenyltrimeticonen und phenylgruppenhaltigen Siliconölen der folgenden Formel (II) 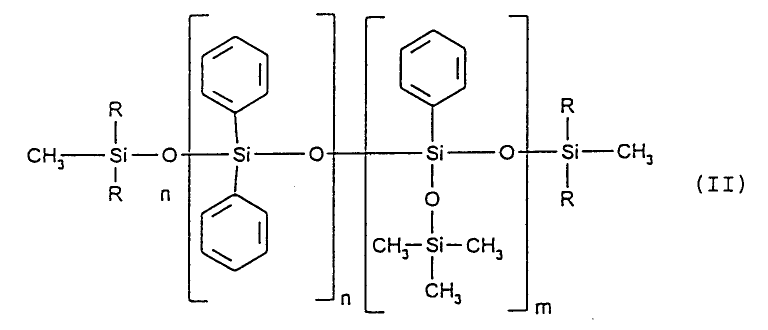 ausgewählt ist, worin bedeuten:
- R eine Alkylgruppe mit 1 bis 30 Kohlenstoffatomen, eine Arylgruppe oder eine Aralkylgruppe,
- n Null oder eine ganze Zahl von 1 bis 100,
- m Null oder eine ganze Zahl von 1 bis 100, mit der Maßgabe, daß m+n im Bereich von 1 bis 100 liegt.

4. Trocknungsmittel nach einem der vorhergehenden Ansprüche, dadurch gekennzeichnet, daß der Gehalt an Siliconöl im Bereich von 1 bis 60 Gew.-%, bezogen auf das Gesamtgewicht des Trocknungsmittels, liegt.

5. Trocknungsmittel nach einem der vorhergehenden Ansprüche, in dem das Siliconöl eine Viskosität von mehr als 20 mPa·s, vorzugsweise von 25 mPa·s bis 10000 mPa·s, aufweist.

6. Trocknungsmittel nach einem der vorhergehenden Ansprüche, dadurch gekennzeichnet, daß der Gehalt an Petrolether im Bereich von 5 bis 99 Gew.-%, bezogen auf das Gesamtgewicht des Trocknungsmittels, liegt.

7. Trocknungsmittel nach einem der vorhergehenden Ansprüche, dadurch gekennzeichnet, daß es außerdem einen Silicongummi enthält.

8. Trocknungsmittel nach Anspruch 7, dadurch gekennzeichnet, daß der Silicongummi, allein oder im Gemisch, unter den Silicongummis der Formel (III) 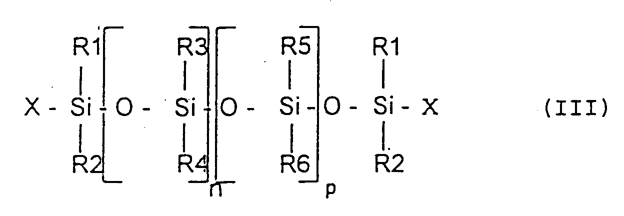 ausgewählt ist, worin bedeuten:
- R₁, R₂, R₅ und R₆, gemeinsam oder unabhängig voneinander, eine Alkylgruppe mit 1 bis 6 Kohlenstoffatomen,
- R₃ und R₄, gemeinsam oder unabhängig voneinander, eine Alkylgruppe mit 1 bis 6 Kohlenstoffatomen oder eine Arylgruppe,
- X eine Alkylgruppe mit 1 bis 6 Kohlenstoffatomen, eine Hydroxygruppe oder eine Vinylgruppe,
wobei
- n und p so ausgewählt sind, daß der Silicongummi eine Viskosität von mehr als 100000 mPa·s hat.

9. Trocknungsmittel nach einem der vorhergehenden Ansprüche, dadurch gekennzeichnet, daß es neben dem Petrolether mindestens eine weitere Verbindung als Lösungsmittel, enthält.

10. Trocknungsmittel nach Anspruch 9, dadurch gekennzeichnet, daß diese weitere Verbindung unter Isopropylmyristat, Isobutan, Isopropanol, Ethanol und deren Gemischen ausgewählt ist.

11. Trocknungsmittel nach einem der vorhergehenden Ansprüche, dadurch gekennzeichnet, daß es mindestens einen Hilfsstoff und/oder kosmetischen Wirkstoff enthält.

12. Verfahren zur Trocknung von Nagellackfilmen, das dadurch gekennzeichnet ist, daß auf den auf dem Nagel gebildeten Film ein Trocknungsmittel nach einem der Ansprüche 1 bis 11 angewendet wird.

13. Verwendung eines Petrolethers und eines Siliconöls in einer kosmetischen Zusammensetzung, die ein Lösungsmittel enthalten kann.

14. Verwendung nach Anspruch 13, um die Trocknung eines Nagellackfilms zu verbessern und/oder zu beschleunigen.
